# EUROPEAN PATENT SPECIFICATION

(11) **EP 3 380 039 B1**
(45) Date of publication and mention of the grant of the patent: **14.09.2022**
(21) Application number: 16867479.4
(22) Date of filing: 22.11.2016
(51) Int. Cl.: A61D 9/00

(54) **EQUINE POULTICE APPLICATION PADS**
PFERDEUMSCHLAGANWENDUNGSPADS
TAMPONS D'APPLICATION DE CATAPLASMES ÉQUINS

(30) Priority: 23.11.2015 US 201562258593 P
(43) Date of publication of application: 03.10.2018
(73) Proprietor: Arion Equine Inc., Flamborough, Ontario L0R 1V0 (CA)
(72) Inventor: QUINN, Anthony, Waterdown, Ontario L0R 2H0 (CA)
(74) Representative: Plasseraud IP
(86) International application number: PCT/CA2016/051369
(87) International publication number: WO 2017/088049

(56) References cited:
- WO-A1-2014/037796
- CA-A1- 2 131 907
- JP-A- 2013 119 025
- US-A1- 2002 056 655
- US-B2- 6 660 901

## Description

### FIELD OF THE INVENTION

The present invention relates generally to the field of animal poultices and, more particularly, to pads or wraps for readily applying poultices to animal limbs, such as horse legs and hooves.

### BACKGROUND OF THE INVENTION

A horse's legs and hooves may require routine attention for swelling and prevention thereof. Commonly, poultices have been used on horses' legs and hooves to treat and relieve inflammation and soreness in the muscles and easily injured tendons in the area, to aid in healing, and/or as a precautionary measure - especially after strenuous riding, running, jumping or competition days, and after other hard work. Typically, all four legs of the horse will be treated at the same time.

A number of commercial poultice mixtures are commercially available as ready-made mixtures for use on horses. These ready-made poultice mixtures typically include mud and/ or clay as the primary active ingredient of the poultice mixture, mixed with water (typically de-ionized water) in known quantities to form a fine, sticky paste-like mixture. The mud and/or clay poultice mixture may desirably comprise, for example, kaolin (white clay) and bentonite (clay made from volcanic ash).

Such ready-made poultice mixtures may additionally comprise, in lesser amounts, various known medicinal and non-medicinal ingredients thought to assist in lessening infection, muscle and tendon swelling and the like, together with known bacteriostatic and mold inhibiting agents for improving the shelf life of the poultice mixture, such as, for example, borax. Most often, a complete list of ready-made poultice mixture ingredients and their proportions is not readily available from producers of these mixtures; rather, such particulars are often treated as a trade secret. One popular poultice mixture suitable for use in the present invention is offered by Wecan Sales Ltd. (of Beamsville, Ontario, Canada) under the product name Uptite^{®}, but the invention is not limited to use with any particular poultice mixture.

Ready-made prior art equine poultice mixtures are typically sold in bulk containers or pails, having a removable and re-sealable lid to facilitate closure between multiple limb applications.

The sticky, paste-like consistency of equine poultice mixtures desirably assists in adhering the poultice mixture to the treatment area of animals, and particularly to the haircovered legs of horses, during the initial step of the traditional four-step poultice application process. However, these same properties, together with the provision of equine poultice mixtures in bulk containers as aforesaid, provide for a potentially messy application scenario, due, at least in part, to the spread of the poultice mixture beyond the intended treatment area of the animal to persons, articles of clothing and other articles in proximity to the intended treatment area during the application process. This spread of the poultice mixture beyond the treatment area of the animal makes for a time-consuming and tedious clean-up process after the poultice application process is completed.

Heretofore, in the first step of the application process, riders and grooms have used rubber gloves to manually apply and pack a layer of poultice mixture onto horses' lower legs to be treated. Thereafter, as the second step, the applied layer of poultice mixture is covered with a piece of sacking, water-moistened paper, paper towel, or similar material to retain desirable levels of moisture within the poultice mixture. Next, as a third step of the poultice application process, a standing bandage may then be wrapped around the manually-applied poultice mixture to compress the mixture and to retain same on the leg of the animal. Finally, as a fourth step of the process, a tensor-type bandage is typically applied over top of the standing bandage to hold the underlying layers firmly in place. Bandages used in the poultice treatment typically may need to be changed every twelve hours.

It will be noted that the prior art application process of such poultices to animals, particularly equine animals, is time-consuming, extremely messy and wasteful of poultice mixture, and/or it is difficult to consistently apply an appropriate amount of poultice mixture evenly over the treatment area.

It is therefore an object of the present invention to provide a poultice application pad that may be used to improve conventional poultice application treatments by making them less time consuming, less messy, and less wasteful of poultice mixture than conventional prior art treatments.

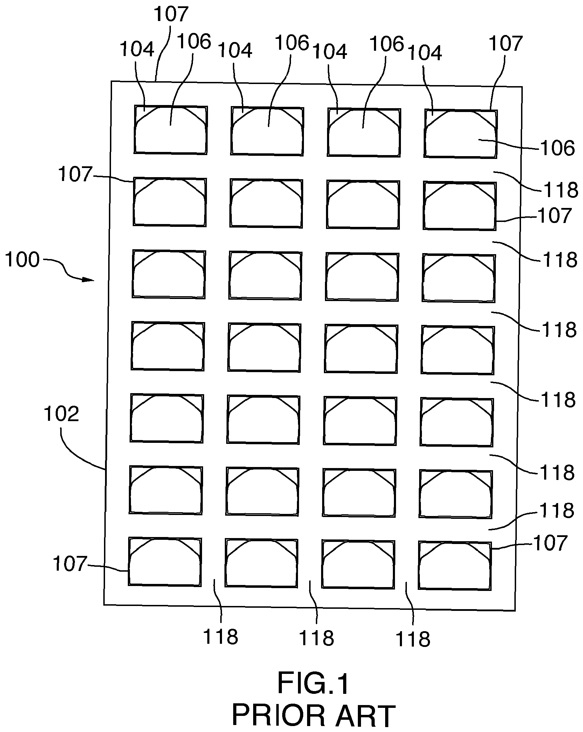

One attempt to address the aforementioned problems and shortcomings with conventional poultice treatments can be seen in bilaminar sheets of quilted fibre paper offered by The Better Bandage Company, Inc. (of Mississauga, Ontario, Canada) under the product name Stayons^{®} for wrapping around horses' legs. International Patent Publication Number WO 2014/037796 A1 entitled "Prefilled Poultice Wraps and Systems" was published on March 13, 2014 in the name of The Better Bandage Company. Figure 1 of the present application depicts a quilted bilaminar fibre paper sheet of the general type disclosed in the aforementioned international publication and offered for sale under the Stayons^{®} product name. Such bilaminar quilted fibre paper has a plurality of closed pockets formed therein by patterned internal fusion of the two layers of the sheet, which pockets are pre-filled to retain dry poultice ingredients (primarily mud and/or clay as aforesaid) therein. Before use, each such sheet of quilted fibre paper containing the dry poultice ingredients must be soaked in water for 15-20 seconds, and thereafter wrapped around an area of the horse's leg to be treated. A standing bandage and/or a tensor-type bandage may then be applied over top of the quilted fibre paper product.

A number of problems remain associated with the use of such bilaminar quilted sheets. For example, in soaking the bilaminar quilted sheets in water, it is difficult to get consistent and/or sufficient water absorption throughout the dry poultice mixture, potentially resulting in areas of variable and/or diminished effectiveness throughout each sheet. Also, the bilaminar quilted sheets necessarily juxtapose a layer of fibre paper between the poultice material and the treatment area of a horse's leg and, as such, the contact between the animal and the poultice material is somewhat diminished and less than with the traditional manually-applied poultice application process describe above, thereby effectively lessening the effectiveness of the treatment. Additionally, the juxtaposed layer of fibre paper in the wet bilaminar quilted sheet will not adhere to the horse's leg as well as a directly exposed moist poultice mixture, such that a user may have to, by hand, hold and maintain the wetted bilaminar quilted sheet in place against the horse's leg while at the same time, if she or he has a free hand, applying a standing bandage and/or a tensor-type bandage over top thereof. This is quite awkward and requires significant manual dexterity on the part of the user, or, alternatively, two persons to more efficiently carry out the process. Moreover, each bilaminar quilted sheet defines numerous gaps in the fused areas between adjacent pockets of poultice mixture, and/or areas of lower poultice concentration within the individual quilted pockets formed by the bilaminar sheet - thereby leaving sections of the treatment area with yet further diminished exposure, or without any exposure whatsoever, to the poultice mixture. Also, the compression provided by the poultice in the quilted pockets may, for the same reason, be uneven.

Other poultice wraps of differing materials are known and made for use on humans, and it is known to combine other structures for compressing or heating areas to be treated. For example, in different fields of art, a portable therapeutic mud/clay applicator is known, wherein a mud/clay therapy plaster layer is protected behind a releasable paper or plastic film, with a nonwoven fabric layer backing behind the mud therapy plaster layer, and a heating pack provided behind the nonwoven fabric layer.

In view of the foregoing, there remains a need for improved manufactures and methods of using same for applying poultice mixtures to the limbs of animals, most notably to the legs of horses. To this end, the present invention provides an improved poultice application pad which does not suffer from one or more of the disadvantages associated with the prior art.

It is an object of the present invention to solve, obviate or mitigate at least one of the problems, disadvantages and/or shortcomings associated with the prior art, to meet or to provide for one or more needs and/or advantages, and/or to achieve one or more objects of the invention - one or more of which may be described hereinabove, or otherwise be readily appreciable by and/or suggested to those skilled in the art in view of the teachings and/or disclosures hereof. Other examples of prior-art can be found for instance in WO 2014/037796 A1 disclosing prefilled poultice wraps, JP 2013/119025 A1 disclosing a poultice set and an applier, US 6660901 B2 disclosing a charcoal skin patch, and CA 2131907 A1 defining storing and dispensing system for products packed in a sealed pouch.

### SUMMARY OF THE INVENTION

According to the invention, there is disclosed a set of poultice application pads as defined in claim 1 and appended claims. At least a back face of the backing sheet is preferably adhesion-resistant to facilitate release and removal from the poultice layer of the adjacent one of the pads, prior to contacting the first side of the poultice layer to the treatment area of the limb of the animal.

According to an aspect of the disclosure the set may include four poultice application pads, preferably one for each limb of an equine animal.

According to an aspect of another embodiment of the invention, each of the poultice application pads in the stack may preferably additionally comprise an adhesion-resistant release sheet mounted atop and adjacent to the first side of the poultice layer of the respective application pad, such that, in the stack, the adhesion-resistant release sheet is juxtaposed between the backing sheet of an upper one of the poultice application pads and the first side of the poultice layer of the adjacent lower one of the poultice application pads. In this manner, the adhesion-resistant properties of the release sheets (i.e., their slipperiness) that contacts the backing sheet of the adjacent poultice application pad facilitates the clean separation and release from the stack of the upper poultice application pad from the adjacent application pad immediately below it in the stack upon removal of each of the upper poultice application pads form the stack. The release sheet will need to be removed from its adherence to the first side of the poultice layer of the uppermost poultice application pad before being brought into contact with the treatment area of the limb of the animal. Accordingly, the release sheet may be advantageously constructed from a paper material, such as parchment paper, wax paper and plastic coated paper, or from a slippery plastics material, such as vinyl, polyethylene or polypropylene.

According to the invention, the poultice application pads are held within a container, preferably, but not essentially, constructed from adhesion-resistant plastic material, in sealed relation from atmosphere to control the escape of moisture from the mud and/or clay mixture while in the container. A set of application pads, typically made up of four application pads, one for application to each limb of a quadruped animal, such as a horse, is stacked one upon the other in the container before sealing of the container. Preferably, the container is vacuum sealed following initial insertion of the pad(s) therein. The container may be a rigid container with a removable, re-sealable lid, or may be a tear-open pouch of flexible foil or plastic film.

The advantages, features and characteristics of the present invention, as well as functions of the related elements of the structure, and the combination of parts and economies of manufacture, will become more apparent upon consideration of the following detailed description of several different embodiments, and the appended claims with reference to the accompanying drawings, the latter of which is briefly described hereinbelow.

### BRIEF DESCRIPTION OF THE DRAWINGS

The novel and inventive features which are believed to be characteristic of the equine poultice application pads according to the present invention, as to its structure, organization, together with further objectives and advantages thereof, will be better understood from the following drawings in which a presently preferred embodiment of the invention will now be illustrated by way of example. In the accompanying drawings:
**Figure 1** is a top plan view of a prior art bi-laminar quilted fibre paper poultice wrap for applying around horses' legs, showing a plurality of pockets pre-filled with a dry poultice mixture, substantially as shown and described in International Patent Publication Number WO 2014/037796 A1 entitled "Prefilled Poultice Wraps and Systems", published March 13, 2014.
**Figure 2A** is a side elevational cross-sectional view of an equine application pad according to a first embodiment of the invention;
**Figure 2B** is a view on an enlarged scale of the encircled area 2B of Figure 2A;
**Figure 3A** is a side elevational cross-sectional view of a stacked set of equine application pads inside a container according to a second embodiment of the invention;
**Figure 3B** is a view on an enlarged scale of the encircled area 3B of Figure 3A;
**Figure 4A** is a side elevational cross-sectional view of a stacked set of equine application pads inside a container according to a third embodiment of the invention;
**Figure 4B** is a is a view on an enlarged scale of the encircled area 4B of Figure 4A;
**Figure 5** is a side elevational cross-sectional view of an equine application pad according to a fourth embodiment of the invention; and
**Figure 6** is a top plan view of the equine application pad of Figure 5, shown after removal of the top adhesion-resistant release sheet thereof for ease of illustrating the poultice layer.

### DETAILED DESCRIPTION OF THE INVENTION

A prior art poultice sheet 100 of bi-laminar quilted fibre paper for applying around a horse's leg is shown in Figure 1 of the drawings. The upper layer of the bi-laminar poultice sheet 100 has been removed in Figure 1 to clearly illustrate the matrix 102 of pockets 107 formed by the quilted sheet, each of which pockets 107 are pre-filled with a dry poultice mixture as disclosed more fully in WO 2014/037796. These quilted fibre paper sheets 100 must be soaked in water before being wrapped around an area of the horse's leg to be treated. Problematically, it is difficult to get consistent and/or sufficient water absorption throughout the dry poultice ingredients, which can result in areas of variable and/or diminished effectiveness throughout each sheet 100. Moreover, Figure 1 shows that such quilted sheets 100 define numerous gaps (bands) 118 between adjacent pockets 107 of poultice mixture 106. Problematically, therefore, such a prior art sheet 100 of quilted fibre paper provides for uneven compression of the poultice mixture 106, areas 104 with diminished exposure, or gaps 118 without any exposure whatsoever, of the poultice mixture to the treatment area of the limb of the animal. Additionally, in such prior art quilted sheets 100, an upper layer of fibre paper (not shown in Figure 1) is juxtaposed between the poultice mixture and the area of treatment of the animal, thereby resulting in diminished therapeutic effect over the entire contact area of the sheet 100, as compared to a poultice mixture directly contacting the epidermis of the animal in the treatment area. Moreover, the presence of such an upper layer (not shown) covering the otherwise exposed sticky outer surface of the poultice mixture, results in reduced adherence of the poultice mixture to the treatment area, such that it may require a user to, with one hand, hold and maintain the wet quilted sheet 100 in place against the animal's leg while, at the same time, attempting to apply with the user's other hand, a standing bandage and/or a tensor-type bandage over the quilted sheet 100. Such an operation may, without additional help, be awkward, time consuming and frustrating.

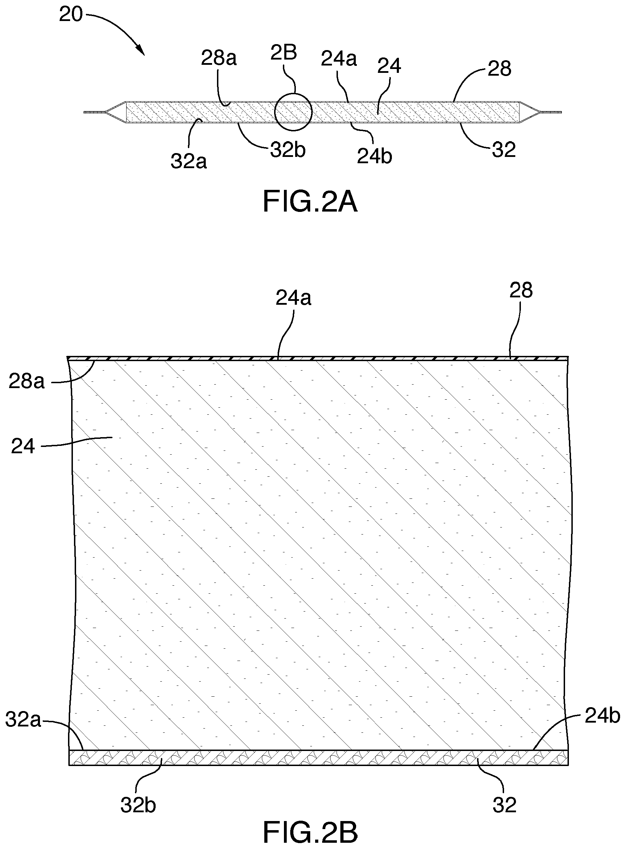

Referring now to Figures 2A and 2B of the drawings, there is shown a poultice application pad 20 according to a first embodiment of the invention for application to a treatment area of an animal(not shown),such as, for example, the lower leg area of a horse. The poultice application pad 20 necessarily includes a generally planar poultice layer 24, having a first side 24a and a second side 24b and a backing sheet 32 positioned in contact with the second side 24b of the poultice layer 24 to support and hold the form of the poultice application layer 24 when contacting the first side 24a of the poultice layer 24 to the treatment area of the animal. An optional, but preferably included, adhesion-resistant release sheet 28 selectively contacts the first side 24a of the poultice layer 24 and is removable from such contact before application of the first side of the 24a of the poultice layer to the treatment area.

The poultice layer 24 comprises a conventional mud and/or clay poultice mixture, which mixture may, as discussed above, include kaolin (white clay) and/or bentonite (clays made from volcanic ash) and may additionally comprise, in lesser amounts, various known medicinal and non-medicinal ingredients such as Epsom salt (magnesium sulfate) thought to assist in lessening infection, muscle and tendon swelling and the like, emulsifiers such as guar gum for maintaining the texture of the poultice mixture, together with other known bacteriostatic and mold inhibiting agents for improving the shelf life of the poultice mixture, such as, for example, borax. As previously mentioned, one such commercially-available poultice suitable for use in the poultice layer 24, according to the present invention is the Uptite^{®} poultice mixture offered by Wecan Sales Ltd. (of Beamsville, Ontario, Canada).

The backing sheet 32, as seen in Figures 2A and 2B, has an upper surface 32a and a lower surface 32b, with the upper surface 32a in contact with the second side 24b of the poultice layer 24. The backing sheet 32 may be constructed from a paper material, such as, for example, butcher's paper, parchment paper, paper towel paper or wax paper. Especially useful for construction of the backing sheet 32 is the paper towel material used in the manufacture of blue-colored paper shop towels which are available from Kimberly-Clark Global Sales, LLC Inc. of Roswell, Georgia, USA under the trademark SCOTT SHOP TOWELS^{™}. With this material, whose precise formulation is proprietary, the upper surface 32a readily adheres to the second (lower) side 24b of the poultice layer 24, which adherence is an important characteristic in selecting the material of the backing sheet 32. Where, for example, a material such as butcher's paper is used to construct the backing sheet 32, (which paper has a dull, more-adherent first side and a shiny less-adherent second side), the shiny less-adherent second side should be arranged to face outwardly (i.e., downwardly in the Figures) away from the second (lower) side 24b of the poultice lawyer, with the dull more-adherent first side arranged to face inwardly (i.e., upwardly in the Figures) to contact the second side 24b of the poultice layer. The blue-colored paper shop towel material is also preferred as a material for construction of the backing layer 32 as it retains moisture well and is considerably more tearresistant than normal paper towel material. Moreover, it readily conforms during application to the shape of the leg of the animal being treated.

Alternatively, the backing sheet 32 may be constructed from a woven fabric material, such as, for example cheesecloth, burlap, gauze and gauze-like cotton cloth. As such, the backing sheet 32 supports and holds the form of the poultice layer 24, and also assists in preventing the poultice mixture from drying out prematurely during the period of 12 to 15 hours during which it is typically applied to the leg of the animal being treated.

By supporting and holding the form of the poultice layer 24 during application of the poultice mixture to the treatment area, the backing sheet 32 is instrumental in containing the poultice mixture proximate to the application area during the application process, thereby significantly reducing the mess and waste caused by poultice mixture that is displaced beyond the treatment area during conventional poultice application procedures.

As seen before use in Figures 2A and 2B, the adhesion-resistant release sheet 28 of the equine poultice application pad 20 contacts, by its lower side 28a, the first side 24a of the poultice layer 24. In this configuration, the release sheet 28 protects the poultice layer from drying out before use, and assists in stabilizing and holding the form of the poultice layer 24 while the application pad 20 is in transport or storage. Just prior to being used, the release sheet 28 is torn away, or otherwise removed from, the aforesaid contact with the first side 24a of the poultice layer 24, so that the first side 24a of the poultice layer 24 may thereafter be directly contacted with the treatment area of the animal.

The adhesion-resistant release sheet 28 may be constructed from a paper material, such as, for example, butcher's paper (shiny side down), parchment paper, wax paper, or plastic coated paper. Preferably, the release sheet 28 is constructed from a adhesion-resistant plastic material, such as vinyl, polyethylene or polypropylene, so as to retain moisture in the poultice layer 24 during transport and storage and so as to resist sticking of the release sheet to the first side 24a of the poultice layer 24 when the release sheet 28 is torn away from the poultice layer 24 by the user prior to application of the pad 20 to the treatment area.

As seen in the embodiment of Figures 2A and 2B, the poultice layer 24 is sandwiched between the backing sheet 32 and the slip-resistant release sheet 28. Preferably, for equine leg wraps, the poultice layer 24 is between about 1/8 to 3/8 of an inch in thickness, and of such width and length to wrap around and cover a portion of the animal's leg to be treated. More preferably, for equine leg wraps, the poultice layer 24 may be between about 1/8 to 1/4 of an inch in thickness.

The adhesion-resistant release sheet 28 and the backing sheet 32 are each preferably dimensioned slightly larger than the width and length of the poultice layer 24, and preferably overlap around all edges of the perimeter of the poultice layer 24 to preferably touch each other as shown in Figure 2A. Such overlapping assists in maintaining moisture within the poultice layer 24 prior to removal of the release sheet 28 and in preventing the poultice mixture from escaping from between the release sheet 28 and the backing sheet 32 before application to the treatment area of the animal.

While prior art poultice mixtures typically have a very sticky, paste-like consistency which allows them to advantageously adhere to the hair and skin of the animal to be treated, it is this same consistency that, less advantageously, makes them stick to most other materials they may come in contact with during the poultice application process, such that cleanup of errant poultice mixture can becomes extremely onerous when poultice mixture is displaced beyond the treatment area of the animal. Thus, use of a backing sheet 32 that adheres to and holds the form of the poultice layer 24 on the backing sheet 32 during application of the pad 20 to the treatment area of the animal significantly lessens transfer of poultice mixture beyond the application area, thereby significantly simplifying and shortening cleanup time over conventional poultice application procedures.

The present invention also significantly simplifies and shortens the time required for a user to carry out the first step of the conventional poultice application process described above, in which conventional first step the user is required to hand pack a layer of poultice mixture onto the leg of the animal by repeatedly scooping of handfuls of the poultice mixture from the bulk containers in which it comes packaged and thereafter smoothing it over the leg of the animal until an acceptable thickness of poultice mixture had been applied to the treatment area. As the optimal quantity of poultice mixture and its thickness is pre-determined during the manufacture of applicant's poultice application pads 20, the present invention not only simplifies this first step of the process by eliminating the trial-and error guesswork required by said first step of the conventional art poultice application process, but also saves time by immediately delivering an appropriate measured amount of poultice mixture in a substantially uniform thickness to the treatment area of the animal coincident with the action of wrapping the poultice application pad 20' around the leg of the animal by a user.

When a poultice is to be applied to a horse's leg, a poultice application pad 20 is retrieved and the adhesion-resistant release sheet 28, (where optionally present), is removed by peeling it away from contact with the poultice layer 24. In this manner, the generally planar first surface 24a of the poultice layer 24 is exposed. The poultice application pad 20 is then applied, poultice layer 24 first, to the area of the horse's leg to be treated, wrapping it therearound with the backing sheet 32 holding the poultice layer 24 in place on the leg of the horse. The exposed poultice layer 24 will stick and adhere to the horse's leg upon contact. Then, without necessarily needing rubber gloves, the user may preferably press on the backing sheet 32 of the poultice application pad 20 to mold it and the poultice layer 24 to substantially every contour and groove of the horse's leg. No added water is required and there will typically be little or no mess or waste caused by displacement of the poultice material beyond the treatment area. Moreover, the poultice application pad 20 may adhere to the horse's leg without needing to be held in place by hand, such that, with her or his hands free, a user may even choose to apply all four poultice application pads 20, one to each of the horse's legs, before proceeding with any secondary bandage wraps. A secondary bandage wrap (*e.g*., a standing bandage) may then be applied around the horse poultice application pad 20 to hold it in place about the area of the horse's leg being treated.

A standing bandage wrap, or stable bandage wrap, is a type of prior art wrap used on the lower legs of a horse. Prior art leg bandage wraps of this type are usually started on the outside of the horse's leg, in the middle of the cannon bone, then wrapped down to the fetlock, then back up to just under the knee, then back to the center of the cannon just above the starting point, ending on the outside of the leg. Most of the time, legs have been wrapped starting on the outside, moving front to back. Such bandage wraps either may be disposable stretchable wrap that sticks to itself, or washable fleece or cotton wraps that are reusable and fasten at the ends with a hook and loop closure or other fasteners. Bandages of this sort may also be taped with medical tape to help them stay on. In this manner, the stable bandage may be used to secure the equine poultice application pad 20 - *i.e*., to hold the equine poultice application pad 20 in place on the lower legs of the animal being treated.

According to other embodiments of the invention, one or more poultice application pads according to the invention may be held either individually, or in a stack, within a container in sealed relation from atmosphere (i.e., in an airtight container) to physically protect the application pads from damage during shipment and/or storage, and to maintain the desired level of moisture within the poultice application pad over extended periods of time following initial manufacture and packaging. Preferably, the container in each of these embodiments is made from an adhesion-resistant plastic material, such as vinyl, polypropylene or polyethylene, so as to provide for non-stick removal of the poultice application pads from the container. The container may be rigid in the form of a tub or the like, with a removable, re-sealable lid, or may be a flexible tear-open foil or plastic film pouch. Most preferably, the container is vacuum sealed at the factory to maintain the level of moisture within the poultice mixture within close tolerances over an extended period of time so as to extend the shelf-life of the poultice application pads packaged therein. While the poultice application pads for some applications may be packaged individually according to this aspect of the invention, preferably, the container is sized and otherwise adapted to hold a stack of four poultice application pads, one for each limb of a quadruped animal, such as a horse.

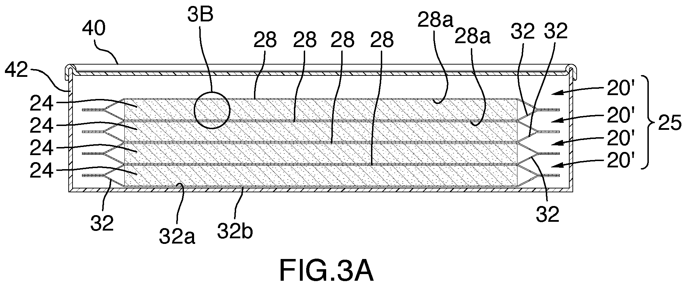

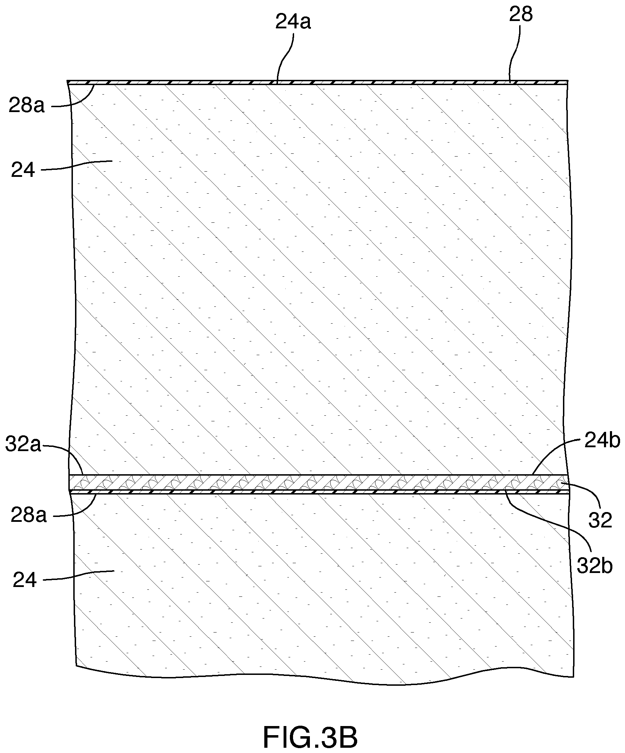

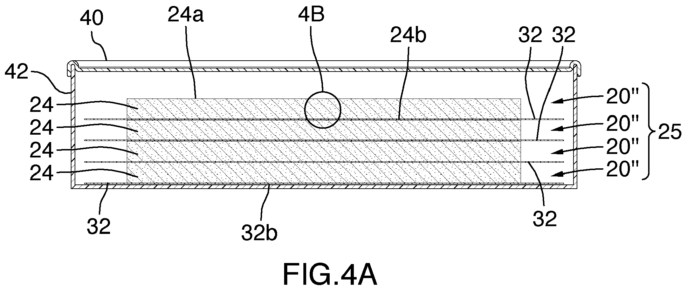

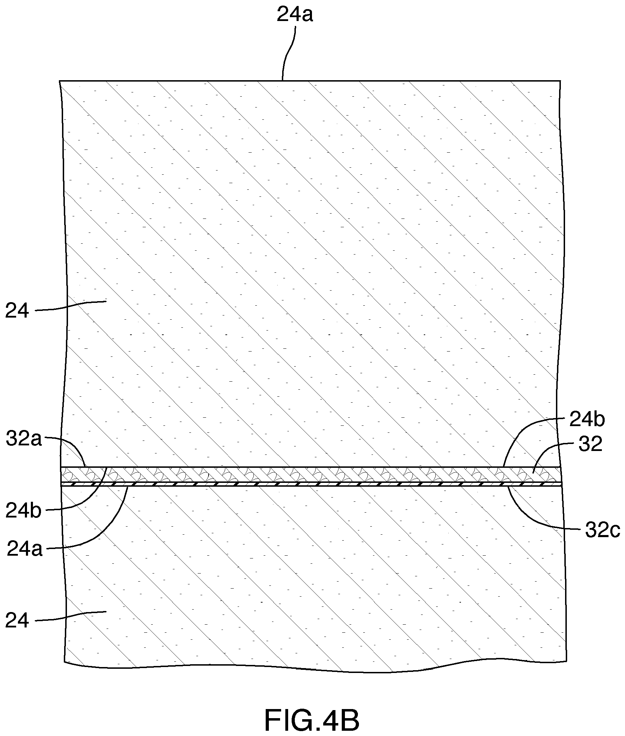

Referring now more particularly to Figures 3A and 3B of the drawings, there will be seen a second embodiment according to the invention wherein an adhesion-resistant plastic container 42 stores a stack of four (4) equine poultice application pads 20', 20', 20', 20' - one for each leg of the horse to be treated. Each of the equine poultice application pads 20' shown in Figures 3A and 3B includes a poultice layer 24, a backing sheet 32, and an adhesion-resistant release sheet 28 of the same general materials and construction as described hereinabove in relation to the first embodiment depicted in Figures 2A and 2B. The adhesion-resistant release sheet 28 associated with each of the equine poultice application pads 20', 20', 20', 20' in the stack is juxtaposed between the backing sheet 32 of an upper one of the poultice application pads 20' and the first side of the poultice layer 24 of an adjacent lower one of the poultice application pads 20' so as to facilitate separation of each of the poultice application pads in the stack one from the other. Without such juxtaposition of the adhesion-resistant release sheet 28 (or other measures such as is described in relation to the third embodiment of Figures 4A and 4B), there would be a tendency for the first side 24a of the poultice layer 24 of a lower poultice application pad 20' in the stack 25 to adhere to lower surface 32b of the adjacent backing sheet 32 of the poultice application pad 20' immediately above it in the stack 25. Thus, the presence of an adhesion-resistant release sheet 28 as a top layer of each of the poultice application pads 20' makes clean and quick separation of the pads 20' from each other easier to achieve with neater results.

Turing to Figures 4A and 4B, there will be seen a third exemplary embodiment of the invention that is the same in all material respects with the second embodiment illustrated in Figures 3A and 3B with one notable exception. That is, in the third embodiment illustrated, there is no adhesion-resistant release sheet 28 provided atop the first surface 24a of any one of the four application pads 20ʺ, 20ʺ, 20ʺ, 20ʺ held within the container 42. Rather, only a backing sheet 32 is provided between adjacent poultice layers 24 in the stack 25 of equine poultice application pads 20", 20", 20", 20". As such, the lower surface 32b of the backing sheet 32 of each poultice application pad 20ʺ shown in the embodiment of Figures 4A and 4B contacts the first side 24a of the poultice layer 24 of each adjacent lower poultice application pad 20ʺ. To compensate for the lack of a release sheet 28, the backing sheet 32 may be coated or treated chemically and/or physically on its lower surface 32b to provide adhesion-resistance to the contacting top surface 24a of the adjacent lower poultice layer in any number of known ways. For example, as shown in Figures 4A and 4B, an adhesion-resistant plastic coating 32c may be applied or otherwise attached to the bottom surface 32b of the backing sheet to provide adhesion resistance to the poultice mixture. In this manner, in the third embodiment of the invention, illustrated the backing sheet 32 acts not only as a support to hold the form of the poultice layer 24 when contacting the first side 24a of the poultice layer 24 to the treatment area, but additionally provides adhesion-resistance to the bottom surface of the backing sheet 32 to facilitate separation of each said upper one of the poultice application pads 20ʺ from each said adjacent lower application pad in the stack 25 upon removal of each said upper poultice application pad from the stack 25. Butcher's paper is also particularly suited for use as a selected material for the construction of the backing sheet 32 of the third embodiment, where the dull, more-adherent upper first surface 32a thereof contacts the second side 24b of the poultice layer 24 of the upper application pad 20ʺ and the shinier, more adhesion-resistant lower second surface 32b thereof contacts the first side 24a of the poultice layer 24 of the adjacent lower application pad 20ʺ immediately therebeneath.

In use, when an equine poultice application pad 20ʺ is required, the container 42 is opened and a top pad 20ʺ is removed by peeling it from the adjacent pad 20ʺ below. An additional protective top sheet (not shown in Figures 4A or 4B) may optionally be employed to further lessen the chances of the topmost poultice layer 24 drying out, or adhering to the undersurface of the cover of the container 42. Ideally, as mentioned above, the container 42 contains four (4) equine poultice applications pads 20', 20', 20', 20' that can be used simultaneously one each or each of the four legs of a horse undergoing a poultice treatment.

In the third embodiment shown in Figures 4A and 4B, the container 42 is substantially the same as the container 42 shown in the second embodiment illustrated in Figures 3A and 3B. Further, the container 42 is ideally also vacuum sealed at the factory prior to shipment therefrom.

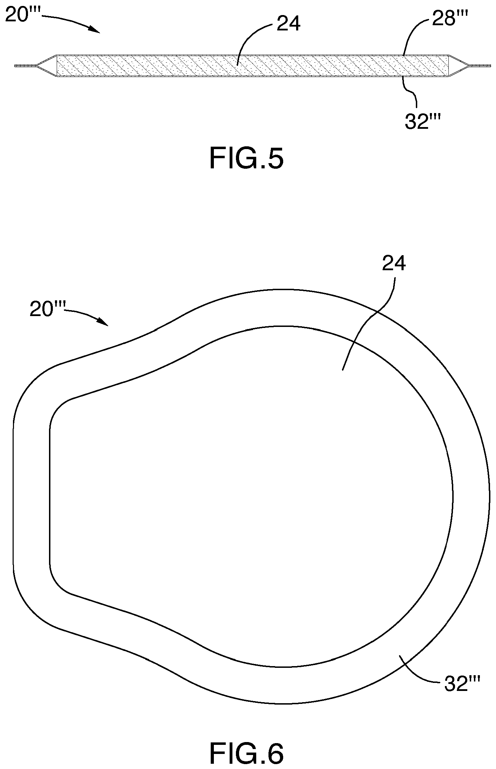

Referring now to Figures 5 and 6 of the drawings, there is shown a fourth embodiment of poultice application pad 20‴ according to the invention, which is preferably for application to the bottom of a horse's hoof. The equine poultice application pad 20‴ shown in Figures 5 and 6 includes a poultice layer 24 and a backing sheet 32ʺ of the same general composition and construction as described hereinabove. It also optionally includes an adhesion-resistant release sheet 28ʺ (although such sheet 28 has been removed and/or is not shown in Figure 6 for ease of illustrating the poultice layer 24).

As best seen in Figure 5, the poultice layer 24 is sandwiched between the adhesion-resistant release sheet 28' and the backing sheet 32'. Preferably, for hoof poultice application pads 20‴, the poultice layer 24 is approximately 1/2 inch to 1 inch thick and of such size and shape to cover the bottom of the hoof to be treated. The adhesion-resistant release sheet 28' and the backing sheet 32' are each preferably dimensioned larger than the width and length of the poultice layer 24, and overlap around the edges of the poultice layer 24 as shown in Figure 5.

The poultice layer 24, adhesion-resistant release sheet 28ʺ and backing sheet 32ʺ of the poultice application pad 20ʺ shown in Figures 5 and 6 may each be formed of materials of the same general type, materials and construction as respectively described hereinabove in the context of the poultice application pads 20 which are shown in Figures 3A and 3B.

The hooves of horses are often treated with poultices on an individual basis, as opposed to horse's legs, which are, in most cases, all four treated simultaneously. Accordingly, while the embodiment of poultice application pad 20‴ illustrated in Figures 5 and 6 may also be sold as a stacked set of four in a container similar in material and structure to the container 42 illustrated in Figures 3A and 4A (only smaller in plan outline), it may by reason of their lower volume usage be more appropriate and desirable to package such application pads 20‴ for hooves individually, whether in solid plastic containers, or in flexible foil or plastic film containers.

When a poultice is to be applied to a horse's hoof, one horse poultice application pad 20‴ as illustrated in Figures 5 and 6 is retrieved and the adhesion-resistant release sheet 28" is removed by peeling it away from the poultice layer 24. In this manner, a generally planar first surface 24a of the poultice layer 24 is exposed. The horse poultice application pad 20‴ is then applied, with the poultice layer 24 first, to the bottom of the horse's hoof to be treated. The poultice layer 24 will stick and adhere to the horse's hoof upon contact. Then, without needing any gloves, a thumb may preferably be used to press on the backing sheet 32ʺ of the poultice application pad 20‴ to pack it tight into the horse's hoof. A secondary wrap (*e.g*., the Vetrap^{™} product offered by the 3M Company of Maplewood, Minnesota, USA) may then applied around the poultice application pad 20‴ to hold it in place on the horse's hoof being treated.

Each of the different poultice application pads 20, 20', and 20ʺ, disclosed hereinabove and each of their respective poultice layers 24 according to the present invention may be provided in small, medium and large sizes to correspond with respective size variations of the horses and other animals on which they may be used.

In the various manners described above, a horse's hooves and one or more, and preferably all four, of a horse's legs may be routinely provided with poultices to treat and prevent swelling and soreness and to aid in healing. Use of the present invention may preferably obviate any need to manually apply poultices in the manner of the prior art, and to reduce the amount of time, mess, and waste of materials which otherwise may be involved when poultices are applied to the legs of a horse. The present invention preferably facilitates and readily provides for a thick, even, direct and consistent application and compression of an appropriate amount of poultice mixture against the legs of an animal, most notably to a horse's legs. The exposed poultice layer of the present invention preferably sticks and adheres to the horse's leg upon contact. The present invention preferably does away with any need for gloves (or added water), and minimizes mess and waste, while affording users with an ability mold the poultice application pad to substantially every contour and groove of the horse's leg or hoof. The present invention preferably frees up a user's hands and even affords an ability to apply all four poultice application pads, one to each of the horse's legs, before proceeding with any secondary bandage wraps. Accordingly, the present invention preferably provides improved equine poultice application pads, and an improved method of applying poultice to a limb of an animal, such as a horse. The equine poultice application pads or wraps of the present invention preferably do not suffer from one or more of the disadvantages which may have been associated with the prior art. Accordingly, the present invention preferably solves, obviates or mitigates one or more problems, disadvantages or shortcomings associated with the prior art, or meets or provides for one or more needs or advantages.

Notwithstanding the above description in association with horses, persons skilled in the art should readily appreciate (in view of the disclosures herein) that the above described equine poultice application pads 20, 20', 20ʺ, and 20‴ can be readily adapted for use on any animal.

Other modifications and alterations may be used in the design and manufacture of other embodiments according to the present invention without departing from the scope of the invention, which is limited only by the accompanying claims.

## Claims

1. A set of poultice application pads (20), wherein each of the poultice application pads (20) comprises:
(A) a generally planar poultice layer (24) comprising a mud and/or clay poultice mixture for application to a treatment area of a limb of an animal, said poultice layer (24) having first and second sides (24a,24b);
(B) at least one backing sheet (32), adhered to the second side (24b) of the poultice layer (24), which backing sheet (32) is adapted for surrounding said limb and to hold the form of the poultice layer (24) when contacting the first side (24a) of the poultice layer to the treatment area to reduce application time and displacement of the mud and/or clay mixture beyond the treatment area;
**characterized in that** the poultice application pads (20) are provided in a stack (25), one on top of another, with the backing sheet (32) of an adjacent upper one of the poultice application pads (20) overlying the first side (24a) of the poultice layer (24) of an adjacent lower one of the poultice application pads (20).

2. The set of poultice application pads (20) of claim 1, wherein each of the poultice application pads (20) further comprises:
(C) an adhesion-resistant release sheet (28), covering the first side of the poultice layer (24a);
wherein, in the stack (25), the adhesion-resistant release sheet (28) is juxtaposed between the backing sheet (32) of an upper one of the poultice application pads (20) and the first side (24a) of the poultice layer (24) of an adjacent lower one of the poultice application pads (20) so as to facilitate separation of each said one of the upper poultice application pads (20) from each of said adjacent lower poultice application pads (20) in the stack (25) upon removal of each said upper poultice application pad (20) from the stack (25).

3. The set of poultice application pads (20) of claim 1, wherein in the stack (25), the backing sheet (32) of an upper one of the poultice application pads (20) contacts the first side of the poultice layer (24a) of each adjacent lower poultice application pad (20); and wherein at least a lower surface (32b) of each backing sheet (32) is adhesion-resistant to facilitate separation of each said upper poultice application pad (20) from each said adjacent lower application pad (20) in the stack (25) upon removal of each said upper poultice application pad (20) from the stack (25).

4. The set of poultice application pads (20) of any one of claims 1 to 3, wherein the set is held within a container (42) in sealed relation from atmosphere to control the escape of moisture from said mud and/or clay mixture while in said container (42) .

5. The set of poultice application pads (20) of claim 4, wherein the set is held in vacuum-sealed relation within said container (42).

6. The set of poultice application pads (20) of any one of claims 4 to 5, wherein the container (42) is formed from an adhesion-resistant plastic material adapted for non-stick removal of the poultice application pads (20) therefrom.

7. The set of poultice application pads (20) of claim 6, wherein the container (42) is a tear-open pouch.

8. The set of poultice application pads (20) of claim 6, wherein the container is a rigid container with a removable, re-sealable lid (40) .

9. The set of poultice application pads (20) of any one of claims 1 to 8, wherein in each of the equine poultice application pads (20), the backing sheet (32) is constructed from a paper material.

10. The set of poultice application pads (20) of any one of claims 1 to 8, wherein the backing sheet (32) of each of the poultice application pads (20) is constructed from a woven fabric material.

11. The set of poultice application pads (20) of any one of claims 3 to 10, wherein the adhesion-resistant release sheet (28) of each poultice application pad (20) is constructed from a paper material.

12. The set of poultice application pads (20) of any one of claims 3 to 11, wherein each of the adhesion-resistant release sheets (28) is constructed from an adhesion-resistant plastic material.

13. The set of poultice application pads (20) according to any one of claims 3 to 12, wherein, in each of the poultice application pads (20), the mud and/or clay mixture includes kaolin.

14. The set of poultice application pads (20) according to any one of claims 3 to 13 wherein, in each of the poultice application pads (20), the mud and/or clay mixture comprises includes bentonite.

## Patentansprüche

1. Satz von Umschlaganwendungspads (20), wobei jedes der Umschlaganwendungspads (20) aufweist:
(A) eine im Allgemeinen ebene Umschlagschicht (24), die eine Schlamm- und/oder Lehmumschlagmischung zum Aufbringen auf einen Behandlungsbereich eines Körperglieds eines Tieres aufweist, wobei die Umschlagschicht (24) eine erste und eine zweite Seite (24a, 24b) aufweist;
(B) mindestens eine Trägerlage (32), die an der zweiten Seite (24b) der Umschlagschicht (24) haftet, wobei die Trägerlage (32) dazu geeignet ist, das Körperglied zu umgeben und die Form der Umschlagschicht (24) zu halten, wenn die erste Seite (24a) der Umschlagschicht mit dem Behandlungsbereich in Kontakt gebracht wird, um die Anwendungszeit und die Verschiebung der Schlamm- und/oder Lehmmischung über den Behandlungsbereich hinaus zu reduzieren;
**dadurch gekennzeichnet, dass** die Umschlaganwendungspads (20) in einem Stapel (25) übereinander vorgesehen sind, wobei die Trägerlage (32) eines benachbarten oberen der Umschlaganwendungspads (20) über der ersten Seite (24a) der Umschlagschicht (24) eines benachbarten unteren der Umschlaganwendungspads (20) liegt.

2. Satz von Umschlaganwendungspads (20) nach Anspruch 1, wobei jede der Umschlaganwendungspads (20) ferner aufweist:
(C) eine haftungsbeständige Trennlage (28), die die erste Seite der Umschlagschicht (24a) bedeckt;
wobei im Stapel (25) die haftungsbeständige Trennlage (28) zwischen der Trägerlage (32) eines oberen der Umschlaganwendungspads (20) und der ersten Seite (24a) der Umschlagschicht (24) eines benachbarten unteren der Umschlaganwendungspads (20) angeordnet ist, um die Trennung jedes der oberen Umschlaganwendungspads (20) von jedem der benachbarten unteren Umschlaganwendungspads (20) im Stapel (25) beim Entfernen jedes oberen Umschlaganwendungspads (20) vom Stapel (25) zu erleichtern.

3. Satz von Umschlaganwendungspads (20) nach Anspruch 1, wobei im Stapel (25) die Trägerlage (32) eines oberen der Umschlaganwendungspads (20) die erste Seite der Umschlagschicht (24a) jedes benachbarten unteren Umschlaganwendungspads (20) berührt; und wobei mindestens eine Unterseite (32b) jeder Trägerlage (32) haftungsbeständig ist, um die Trennung jedes oberen Umschlaganwendungspads (20) von jedem benachbarten unteren Anwendungspad (20) im Stapel (25) beim Entfernen jedes oberen Umschlaganwendungspads (20) vom Stapel (25) zu erleichtern.

4. Satz von Umschlaganwendungspads (20) nach einem der Ansprüche 1 bis 3, wobei der Satz in einem Behälter (42) in abgedichteter Beziehung zur Atmosphäre gehalten wird, um das Entweichen von Feuchtigkeit aus dem Schlamm- und/oder Tongemisch zu kontrollieren, während es sich in dem Behälter (42) befindet.

5. Satz von Umschlaganwendungspads (20) nach Anspruch 4, wobei der Satz innerhalb des Behälters (42) vakuumversiegelt gehalten wird.

6. Satz von Umschlaganwendungspads (20) nach einem der Ansprüche 4 bis 5, wobei der Behälter (42) aus einem haftungsbeständigen Kunststoffmaterial besteht, das zur nicht klebenden Entnahme der Umschlaganwendungspads (20) daraus geeignet ist.

7. Satz von Umschlaganwendungspads (20) nach Anspruch 6, wobei der Behälter (42) ein aufreißbarer Beutel ist.

8. Satz von Umschlaganwendungspads (20) nach Anspruch 6, wobei der Behälter ein starrer Behälter mit einem abnehmbaren, wiederverschließbaren Deckel (40) ist.

9. Satz von Umschlaganwendungspads (20) nach einem der Ansprüche 1 bis 8, wobei in jedem der Umschlaganwendungspads (20) für Pferde die Trägerlage (32) aus einem Papiermaterial aufgebaut ist.

10. Satz von Umschlaganwendungspads (20) nach einem der Ansprüche 1 bis 8, wobei die Trägerlage (32) von jedem der Umschlaganwendungspads (20) aus einem gewebten Stoffmaterial aufgebaut ist.

11. Satz von Umschlaganwendungspads (20) nach einem der Ansprüche 3 bis 10, wobei die haftungsbeständige Trennlage (28) jedes Umschlaganwendungspads (20) aus einem Papiermaterial aufgebaut ist.

12. Satz von Umschlaganwendungspads (20) nach einem der Ansprüche 3 bis 11, wobei jede der haftungsbeständige Trennlagen (28) aus einem haftungsbeständigen Kunststoffmaterial aufgebaut ist.

13. Satz von Umschlaganwendungspads (20) nach einem der Ansprüche 3 bis 12, wobei in jedem der Umschlaganwendungspads (20) die Schlamm- und/oder Lehmmischung Kaolin aufweist.

14. Satz von Umschlaganwendungspads (20) nach einem der Ansprüche 3 bis 13, wobei in jeder der Umschlaganwendungspads (20) die Schlamm- und/oder Lehmmischung Bentonit aufweist.

## Revendications

1. Ensemble de tampons d'application de cataplasme (20), dans lequel chacun des tampons d'application de cataplasme (20) comprend :
(A) une couche de cataplasme (24) généralement plane comprenant un mélange de cataplasme de boue et/ou d'argile pour application à une zone de traitement d'un membre d'un animal, ladite couche de cataplasme (24) ayant des premier et second côtés (24a, 24b) ;
(B) au moins une feuille doublure (32), collée au second côté (24b) de la couche de cataplasme (24), laquelle feuille doublure (32) est adaptée pour entourer ledit membre et pour maintenir la forme de la couche de cataplasme (24) lors du contact du premier côté (24a) de la couche de cataplasme avec la zone de traitement pour réduire un temps d'application et un déplacement du mélange de boue et/ou d'argile au-delà de la zone de traitement ;
**caractérisé en ce que** les tampons d'application de cataplasme (20) sont prévus dans une pile (25), l'un au-dessus de l'autre, la feuille doublure (32) d'un tampon supérieur adjacent des tampons d'application de cataplasme (20) chevauchant le premier côté (24a) de la couche de cataplasme (24) d'un tampon inférieur adjacent des tampons d'application de cataplasme (20).

2. Ensemble de tampons d'application de cataplasme (20) selon la revendication 1, dans lequel chacun des tampons d'application de cataplasme (20) comprend en outre :
(C) une feuille détachable antiadhésive (28), couvrant le premier côté (24a) de la couche de cataplasme ;
dans lequel, dans la pile (25), la feuille détachable antiadhésive (28) est juxtaposée entre la feuille doublure (32) d'un tampon supérieur des tampons d'application de cataplasme (20) et le premier côté (24a) de la couche de cataplasme (24) d'un tampon inférieur adjacent des tampons d'application de cataplasme (20) de façon à faciliter la séparation de chaque dit un des tampons d'application de cataplasme (20) supérieurs de chacun desdits tampons d'application de cataplasme (20) inférieurs adjacents dans la pile (25) lors du retrait de chaque dit tampon d'application de cataplasme (20) supérieur de la pile (25).

3. Ensemble de tampons d'application de cataplasme (20) selon la revendication 1, dans lequel dans la pile (25), la feuille doublure (32) d'un tampon supérieur des tampons d'application de cataplasme (20) est en contact avec le premier côté de la couche de cataplasme (24a) de chaque tampon d'application de cataplasme (20) inférieur adjacent ; et dans lequel au moins une surface inférieure (32b) de chaque feuille doublure (32) est antiadhésive pour faciliter la séparation de chaque dit tampon d'application de cataplasme (20) supérieur de chaque dit tampon d'application (20) inférieur adjacent dans la pile (25) lors du retrait de chaque dit tampon d'application de cataplasme (20) supérieur de la pile (25).

4. Ensemble de tampons d'application de cataplasme (20) selon l'une quelconque des revendications 1 à 3, dans lequel l'ensemble est maintenu à l'intérieur d'un contenant (42) en relation étanche à l'atmosphère pour contrôler l'échappement de l'humidité dudit mélange de boue et/ou d'argile pendant qu'il est dans ledit contenant (42).

5. Ensemble de tampons d'application de cataplasme (20) selon la revendication 4, dans lequel l'ensemble est maintenu en relation scellée sous vide à l'intérieur dudit contenant (42).

6. Ensemble de tampons d'application de cataplasme (20) selon l'une quelconque des revendications 4 et 5, dans lequel le contenant (42) est formé à partir d'un matériau plastique antiadhésif adapté pour un retrait non collant des tampons d'application de cataplasme (20) de celui-ci.

7. Ensemble de tampons d'application de cataplasme (20) selon la revendication 6, dans lequel le contenant (42) est une poche à ouverture par déchirement.

8. Ensemble de tampons d'application de cataplasme (20) selon la revendication 6, dans lequel le contenant est un contenant rigide avec un couvercle amovible et refermable de manière étanche (40).

9. Ensemble de tampons d'application de cataplasme (20) selon l'une quelconque des revendications 1 à 8, dans lequel dans chacun des tampons d'application de cataplasme (20) équins, la feuille doublure (32) est fabriquée à partir d'un matériau en papier.

10. Ensemble de tampons d'application de cataplasme (20) selon l'une quelconque des revendications 1 à 8, dans lequel la feuille doublure (32) de chacun des tampons d'application de cataplasme (20) est fabriquée à partir d'un matériau de tissu tissé.

11. Ensemble de tampons d'application de cataplasme (20) selon l'une quelconque des revendications 3 à 10, dans lequel la feuille détachable antiadhésive (28) de chaque tampon d'application de cataplasme (20) est fabriquée à partir d'un matériau en papier.

12. Ensemble de tampons d'application de cataplasme (20) selon l'une quelconque des revendications 3 à 11, dans lequel chacune des feuilles détachables antiadhésives (28) est fabriquée à partir d'un matériau plastique antiadhésif.

13. Ensemble de tampons d'application de cataplasme (20) selon l'une quelconque des revendications 3 à 12, dans lequel, dans chacun des tampons d'application de cataplasme (20), le mélange de boue et/ou d'argile comporte du kaolin.

14. Ensemble de tampons d'application de cataplasme (20) selon l'une quelconque des revendications 3 à 13 dans lequel, dans chacun des tampons d'application de cataplasme (20), le mélange de boue et/ou d'argile comporte de la bentonite.
